# EUROPÄISCHE PATENTSCHRIFT

(11) **EP 0 594 037 B1**
(45) Veröffentlichungstag und Bekanntmachung des Hinweises auf die Patenterteilung: **24.09.1997**
(21) Anmeldenummer: 93116483.4
(22) Anmeldetag: 12.10.1993
(51) Int. Cl.: C09B 44/02, C07D 211/82

(54) **Basische Farbstoffe auf Basis von Amiden der I-Säure (1-Hydroxy-6-aminonaphthalin-3-sulfonsäure) sowie Amide der I-Säure**
Basic dyes based on amides of I-acid (1-hydroxy-6-aminonaphthalene-3-sulfonic acid) and amides of I-acid
Colorants basiques à base d'amides de l'acide I(acide amino-6 hydroxy-1 naphtalènesulfonique-3) et d'amides de l'acide I

(30) Priorität: 19.10.1992 DE 4235154
(43) Veröffentlichungstag der Anmeldung: 27.04.1994
(73) Patentinhaber: BASF Aktiengesellschaft, 67063 Ludwigshafen (DE)
(72) Erfinder: Schloesser, Ulrike, Dr., D-67071 Ludwigshafen (DE); Mayer, Udo, Dr., D-6710 Frankenthal (DE)

(56) Entgegenhaltungen:
- EP-A- 0 062 824
- EP-A- 0 176 195
- EP-A- 0 212 553
- DE-A- 537 535
- DE-A- 2 724 079
- US-A- 4 363 761
- J.Prakt.Chem.101, 38-57(1921); Römpps Chemie-Lexikon,8.Aufl.,Band 5, S.3438,1987

## Beschreibung

Die vorliegende Erfindung betrifft neue Farbstoffe der Formel I 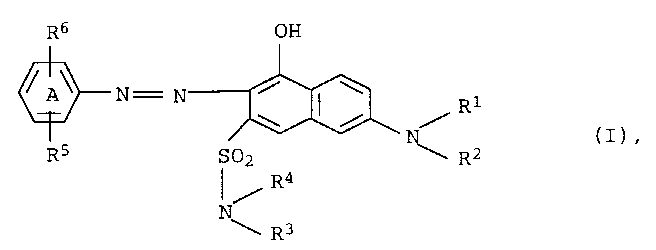 in der
der Ring A benzoanelliert sein kann,
- _{R}1: C₁-C₁₃-Alkyl, Benzyl, 2-Chlorethyl, gegebenenfalls substituiertes Phenyl, C₁-C₈-Alkanoyl, gegebenenfalls substituiertes Benzoyl oder einen Rest der Formel 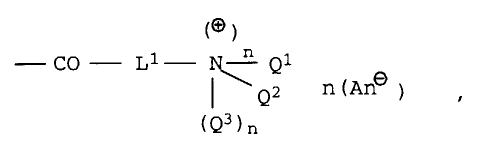 worin L¹ für C₁-C₃-Alkylen,
n für 0 oder 1,
Q¹ Q² Q³ gleich oder verschieden sind und unabhängig voneinander jeweils für Wasserstoff oder gegebenenfalls durch Hydroxy substituiertes C₁-C₈-Alkyl, das durch 1 bis 3 Sauerstoffatome in Etherfunktion unterbrochen sein kann, oder Q¹ und Q² zusammen mit dem sie verbindenden Stickstoffatom für einen 5- oder 6-gliedrigen gesättigten heterocyclischen Rest, der gegebenenfalls Stickstoff oder Sauerstoff als weiteres Heteroatom aufweist, und
An^{⊖} für das Äquivalent eines Anions stehen,
- R²: Wasserstoff oder C₁-C₄-Alkyl oder R¹ und R² zusammen einen Rest der Formel
CO-L² oder CO-L³⁻CO, worin
L² für C₃-C₄-Alkylen und
L3 für C₂-C₃-Alkylen, Vinylen oder 1,2-Phenylen stehen,
- R³ und R⁴: gleich oder verschieden sind und unabhängig voneinander jeweils C₁-C₁₃-Alkyl, das gegebenenfalls durch 1 bis 4 Sauerstoffatome in Etherfunktion oder 1 bis 4 Iminogruppen, die durch C₁-C₄-Alkyl oder ω-Hydroxy-C₁-C₈-alkyl, das gegebenenfalls durch 1 bis 3 Sauerstoffatome in Etherfunktion unterbrochen ist, substituiert sein können, unterbrochen ist und durch Hydroxy,Amino, einen 5- oder 6-gliedrigen gesättigten oder aromatischen heterocyclischen Rest, der ein Stickstoffatom und gegebenenfalls ein weiteres Heteroatom, ausgewählt aus der Gruppe, bestehend aus Stickstoff, Sauerstoff und Schwefel, aufweist, oder gegebenenfalls substituiertes C₅-C₇-Cycloalkyl substituiert sein kann, gegebenenfalls substituiertes Phenyl, gegebenenfalls substituiertes C₅-C₇-Cycloalkyl oder gegebenenfalls durch Methyl substituiertes Piperidinyl oder R³ auch Wasserstoff,
- R⁵: Wasserstoff, C₁-C₄-Alkyl, C₁-C₄-Alkoxy, C₂-C₅-Alkanoylamino, Halogen, Nitro, Cyano, gegebenenfalls durch C₁-C₄-Alkyl substituiertes Phenylazo oder einen Rest der Formel 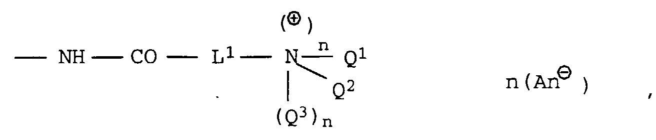 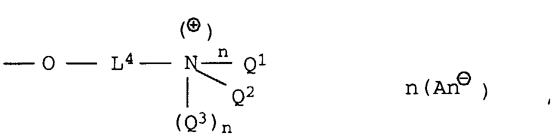 oder 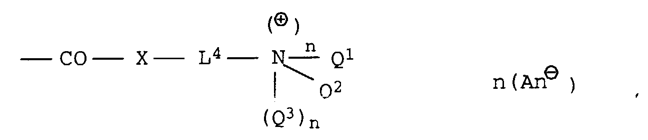 worin X für Sauerstoff oder Imino und L⁴ für C₂-C₄-Alkylen stehen und Q¹, Q², Q³, n und An^{⊖} jeweils die obengenannte Bedeutung besitzen, und
- R⁶: Wasserstoff, C₁-C₄-Alkyl, C₁-C₄-Alkoxy, Halogen, Nitro oder Cyano bedeuten,
mit der Maßgabe, daß mindestens eine quaternierbare Aminogruppe oder eine Ammoniumgruppe im Molekül vorhanden ist, deren Verwendung zum Färben oder Bedrucken von polymerem Material sowie neue Amide der I-Säure.

J. prakt. Chem., Band 101, Seiten 38 bis 57, 1921 beschreibt die 4-Methylphenylazo- und 4-Nitrophenylazoverbindungen von benzoyliertem I-Säurephenylamid. Weiterhin sind aus der US-A-4 363 761 Farbstoffe auf Basis der freien I-Säure bekannt, die Aminotriazinyl- und/oder Carbamoylgruppen aufweisen.

Aufgabe der vorliegenden Erfindung war es, neue basische Azofarbstoffe mit einer Kupplungskomponente aus der Reihe Amide der I-säure(1-Hydroxy-6-aminonaphthalin-3-sulfonsäure) bereitzustellen. Die neuen Farbstoffe sollten sich in vorteilhafter Weise zum Färben oder Bedrucken von polymerem Material, insbesondere von Papier eignen. Die erzielten Färbungen sollten gute Gebrauchseigenschaften aufweisen.

Demgemäß wurden die eingangs näher bezeichneten Farbstoffe der Formel I gefunden.

Alle in der obengenannten Formel auftretenden Alkyl- oder Alkylengruppen können sowohl geradkettig als auch verzweigt sein.

Wenn in der obengenannten Formel substituierte Phenylgruppen auftreten, so können als Substituenten z.B. C₁-C₄-Alkyl, C₁-C₄-Alkoxy oder Halogen, dabei insbesondere Chlor oder Brom, in Betracht kommen. Die Phenylgruppen weisen dann in der Regel 1 bis 3 Substituenten auf.

Wenn in der obengenannten Formel substituierte C₅-C₇-Cycloalkylgruppen auftreten, so können als Substituenten z.B. C₁-C₄-Alkyl oder C₁-C₄-Aminoalkyl in Betracht kommen. Die Cycloalkylgruppen weisen dann in der Regel 1 bis 3 Alkyl- und/ oder eine Aminoalkylgruppe auf.

Wenn in der obengenannten Formel durch Methyl substituiertes Piperidinyl auftritt, so weist es in der Regel 1 bis 4 Methylgruppen auf, wobei substituiertes Piperidin-4-yl bevorzugt ist.

Wenn die Reste R³ oder R⁴ C₁-C₁₃-Alkyl bedeuten, das durch einen 5- oder 6-gliedrigen heterocyclischen Rest, der ein Stickstoffatom und gegebenenfalls ein weiteres Heteroatom, ausgewählt aus der Gruppe, bestehend aus Stickstoff, Sauerstoff und Schwefel, aufweist, substituiert ist, so können als Substituenten gesättigte oder aromatische Reste, wie Pyrrolidinyl, Piperidinyl, Morpholinyl, Piperazinyl, N-(C₁-C₄-Alkyl)piperazinyl, Pyrrolyl, Pyrazolyl, Imidazolyl, Oxazolyl, Isooxazolyl, Thiazolyl oder Isothiazolyl, in Betracht kommen.

Wenn Q¹ und Q² zusammen mit dem sie verbindenden Stickstoffatom für einen 5- oder 6-gliedrigen gesättigten heterocyclischen Rest, der gegebenenfalls Stickstoff oder Sauerstoff als weiteres Heteroatom enthält, steht, so können z.B. Pyrrolidinyl, Piperidinyl, Morpholinyl, Piperazinyl oder N-(C₁-C₄-Methyl)piperazinyl als solche Reste in Betracht kommen.

Wenn in der obengenannten Formel substituierte Alkylreste auftreten, so sind diese in der Regel ein- oder zweifach substituiert.

Reste R¹, R², R³, R⁴, R⁵, R⁶, Q¹, Q² und Q³ sind z.B. Methyl, Ethyl, Propyl, Isopropyl, Butyl, Isobutyl, sec-Butyl oder tert-Butyl.

Reste R¹, R³, R⁴, Q¹, Q² und Q³ sind weiterhin z.B. Pentyl, Isopentyl, Neopentyl, tert-Pentyl, Hexyl, 2-Methylpentyl, Heptyl, Octyl, 2-Ethylhexyl oder Isooctyl.

Reste R¹, R³ und R⁴ sind weiterhin z.B. Nonyl, Isononyl, Decyl, Isodecyl, Undecyl, Dodecyl, Tridecyl, 3,5,5,7-Tetramethylnonyl, Isotridecyl (die obigen Bezeichnungen Isooctyl, Isononyl, Isodecyl und Isotridecyl sind Trivialbezeichnungen und stammen von den nach der Oxosynthese erhaltenen Alkoholen - vgl. dazu Ullmanns Encyklopädie der technischen Chemie, 4. Auflage, Band 7, Seiten 215 bis 217, sowie Band 11, Seiten 435 und 436), Benzyl, 1- oder 2-Phenylethyl, Phenyl, 2-, 3- oder 4-Methylphenyl, 2,4-Dimethylphenyl, 2-, 3- oder 4-Methoxyphenyl, 2,4-Dimethoxyphenyl, 2-, 3- oder 4-Chlorphenyl, oder 2,4-Dichlorphenyl.

Reste R¹ sind weiterhin z.B. Formyl, Acetyl, Propionyl, Butyryl, Isobutyryl, Pentanoyl, Hexanoyl, Heptanoyl, Octanoyl, 2-Ethylhexanoyl, Benzoyl, 2-, 3- oder 4-Methylbenzoyl, 2,4-Dimethylbenzoyl, 2-, 3- oder 4-Methoxybenzoyl, 2,4-Dimethoxybenzoyl, 2-, 3- oder 4-Chlorbenzoyl oder 2,4-Dichlorbenzoyl.

Reste R³ und R⁴ sind weiterhin z.B. 2-Aminoethyl, 2- oder 3-Aminopropyl, 2- oder 4-Aminobutyl, 5-Aminopentyl, 6-Aminohexyl, 7-Aminoheptyl, 8-Aminooctyl, 3-Aza-3-methylbutyl, 4-Aza-4-methylpentyl, 3-Aza-3-ethylpentyl, 4-Aza-4-ethylhexyl, 5-Amino-3-azapentyl, 6-Amino-3-azahexyl, 6-Amino-4-azahexyl, 7-Amino-4-azaheptyl, 8-Amino-3,6-diazaoctyl, 3-Aminoprop-2-yl, 8-Amino-4-(2-aminoethyl)octyl, 2-(Pyrrolidin-1-yl)ethyl, 2- oder 3-(Pyrrolidin-1-yl)propyl, 2-(Piperidin-1-yl)ethyl, 2- oder 3-(Piperidin-1-yl)propyl, 2-(Morpholin-4-yl)ethyl, 2- oder 3-(Morpholin-4-yl)propyl, 2-(Piperazin-1-yl)ethyl, 2- oder 3-(Piperazin-l-yl)propyl, 2-(4-Methylpiperazin-1-yl)ethyl, 2- oder 3-(4-Methylpiperazin-1-yl)propyl, 2-(Imidazol-l-yl)ethyl, 2- oder 3-(Imidazol-1-yl)propyl, Cyclopentyl, Cyclohexyl, Cycloheptyl, 3-Aminomethyl-3,5,5-trimethylcyclohexyl oder 2,2,6,6-Tetramethylpiperidin-4-yl.

Reste R^{3,} R⁴, Q¹, Q² und Q³ sind weiterhin z.B. 2-Methoxyethyl, 2-Ethoxyethyl, 2-Propoxyethyl, 2-Isopropoxyethyl, 2-Butoxyethyl, 2- oder 3-Methoxypropyl, 2- oder 3-Ethoxypropyl, 2- oder 3-Propoxypropyl, 2- oder 3-Butoxypropyl, 2- oder 4-Methoxybutyl, 2- oder 4-Ethoxybutyl, 2- oder 4-Propoxybutyl, 3,6-Dioxaheptyl, 3,6-Dioxyoctyl, 4,8-Dioxanonyl, 3,7-Dioxaoctyl, 3,7-Dioxanonyl, 4,7-Dioxaoctyl, 4,7-Dioxanonyl, 2- oder 4-Butoxybutyl, 4,8-Dioxadecyl, 3,6,9-Trioxadecyl oder 3,6,9-Trioxaundecyl.

Reste R³ und R⁴ sind weiterhin z.B. 3,6,9-Trioxadodecyl, 3,6,9,12-Tetraoxatridecyl, 3,6,9,12-Tetraoxatetradecyl, 4-Hydroxy-2-methyl-3-azabutyl, 4-Hydroxy-3-hydroxymethyl-2-methyl-3-azabutyl, 5-Hydroxy-2-methyl-3-azapentyl, 5-Hydroxy-3-(2-hydroxyethyl)-2-methyl-3-azapentyl, 8-Hydroxy-2-methyl-3-aza-6-oxaoctyl, 11-Hydroxy-2-methyl-3-aza-6,9-dioxaundecyl, 8-Hydroxy-3-(5-hydroxy-3-oxapentyl)-2-methyl-3-aza-6-oxaoctyl oder 11-Hydroxy-3-(8-hydroxy-3,6-dioxaoctyl)-2-methyl-3-aza-6,9-dioxaundecyl.

Reste R⁵ und R⁶ sind weiterhin z.B. Methoxy, Ethoxy, Propoxy, Isopropoxy, Butoxy, Isobutoxy, sec-Butoxy, Fluor, Chlor oder Brom.

Reste R⁵ sind weiterhin z.B. Acetylamino, Propionylamino, Butyrylamino, Isobutyrylamino, Pentanoylamino, Phenylazo oder 2-, 3- oder 4-Methylphenylazo.

Reste R³, R⁴, Q¹, Q² und Q³ sind weiterhin z.B. 2-Hydroxyethyl, 2- oder 3-Hydroxyethyl oder 2- oder 4-Hydroxybutyl.

Reste L¹ sind z.B. Methylen, Ethylen oder 1,2- oder 1,3-Propylen.

Reste L² sind z.B. 1,2- oder 1,3-Propylen oder 1,2-, 2,3- oder 1,4-Butylen.

Reste L³ sind z.B. Ethylen oder 1,2- oder 1,3-Propylen.

Das Äquivalent An^{⊖} eines Anions leitet sich z.B. von folgenden Anionen ab: Fluorid, Chlorid, Bromid, Iodid, Formiat, Acetat, Propionat, Mono-, Di- oder Trichloracetat, Lactat, Methoxyacetat, Citrat, Succinat, Methansulfonat, Benzolsulfonat, 2- oder 4-Methylbenzolsulfonat oder Naphthalinsulfonat.

Bevorzugt sind Farbstoffe der Formel I in der R¹, C₂-C₄-Al-kanoyl, Benzoyl oder einen Rest der Formel 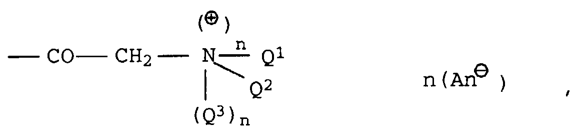 worin Q¹, Q², Q³, An^{⊖} und n jeweils die obengenannte Bedeutung besitzten, oder R¹ und R² zusammen für einen Rest der Formel CO-L³-CO, worin L³ für Ethylen, Vinylen oder 1,2-Phenylen steht und R² Wasserstoff bedeuten.

Bevorzugt sind weiterhin Farbstoffe der Formel I, in der
- R³: Wasserstoff und
- R⁴: C₁-C₁₃-Alkyl bedeuten, das durch 1 oder 2 Sauerstoffatome in Etherfunktion, Imino- oder C₁-C₄-Alkyliminogruppen unterbrochen ist und/oder durch Amino oder einen 5- oder 6-gliedrigen gesättigten oder aromatischen heterocyclischen Rest, der ein oder zwei Stickstoffatome aufweist, substituiert ist.

Besonders bevorzugt sind Farbstoffe der Formel I, in der R¹ Acetyl bedeutet.

Bevorzugt sind weiterhin Farbstoffe aus Formel I in der R⁵ C₁-C₄-Alkyl, C₁-C₄-Alkoxy, C₂-C₄-Alkanoylamino oder Phenylazo und R⁶ Wasserstoff bedeuten.

Die neuen basischen Azofarbstoffe der Formel I können nach an sich bekannten Methoden erhalten werden.

Beispielsweise kann ein Anilin der Formel II 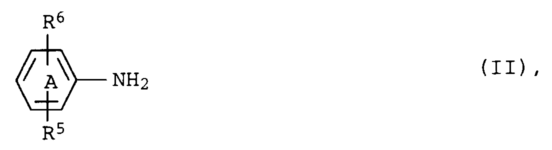 in der R⁵, R⁶ und der Ring A jeweils die obengenannte Bedeutung besitzen, auf an sicn bekanntem Weg diazotiert und mit einem Naphthalinderivat der Formel III 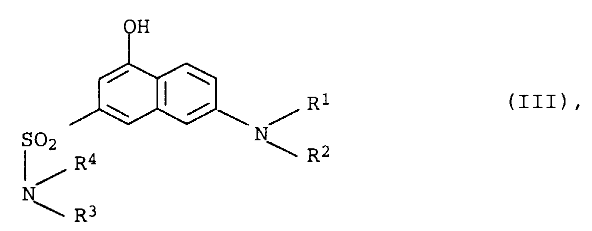 in der R¹, R², R³ und R⁴ jeweils die obengenannte Bedeutung besitzen, gekuppelt werden.

Diejenigen Farbstoffe der Formel I, die über einen quartären Rest verfügen, werden zweckmäßig so erhalten, daß man zunächst den neutralen Azofarbstoff herstellt und diesen dann anschließend mit einem C₂-C₄-Alkylenoxid oder mit einer Verbindung der Formel IV

Q⁴-Y (IV),

in der Q⁴ gegebenenfalls durch Hydroxy substituiertes C₁-C₄-Alkyl und Y eine Austrittsgruppe, z.B. Chlorid, Bromid, Iodid, Methosulfat, Ethosulfat, Benzolsulfonat oder 2- oder 4-Methylbenzolsulfonat, bedeuten, quaterniert.

Einige der Naphthalinderivate der Formel III sind an sich bekannt und z.B. in der EP-A-537 535 beschrieben.

Die erfindungsgemäßen basischen Azofarbstoffe der Formel I können für sich alleine, in Gemischen untereinander und zusammen mit anderen kationischen oder anionischen Verbindungen in Form ihrer Lösungen oder in Form von Pulvern oder Granulaten angewendet werden. Sie eignen sich vorteilhaft zum Färben oder Bedrucken von polymerem Material, insbesondere von Papierstoffen, aber auch von Cellulose, Baumwolle, Leder, Bastfasern, Hanf, Flachs, Sisal, Jute, Kokos oder Stroh.

Bei der Herstellung von Farbstoffpräparationen, enthaltend die neuen Farbstoffe der Formel I, ist die Anwendung von Polymeren, wie Polyacrylsäuren, Polyacrylsäurederivaten, Polyvinylaminen, Polyvinylamiden, Polyvinylacetaten, Polyvinylalkoholen, Polyvinylpyrrolidonen, Polysiloxanen oder Copolymeren der jeweiligen Monomeren hervorzuheben. Desgleichen können Oligomere des Ethylenimins, Ethylenoxids oder Propylenoxids oder Derivate dieser Oligomeren zur Anwendung kommen.

Die Farbstoffe können vorzugsweise bei der Herstellung von in der Masse gefärbtem, geleimtem und ungeleimtem Papier verwendet werden. Sie können ebenfalls zum Färben von Papier nach dem Tauchverfahren angewendet werden.

Das Färben von Papier, Leder oder Cellulose erfolgt nach an sich bekannten Methoden.

Die neuen Farbstoffe oder ihre Präparationen färben das Abwasser bei der Papierherstellung praktisch gar nicht oder nur wenig an, was für die Reinhaltung der Gewässer besonders günstig ist. Sie sind hoch substantiv, melieren nicht, wenn sie auf Papier gefärbt sind und sind weitgehend pH-unempfindlich. Die Färbungen auf Papier zeichnen sich durch eine gute Lichtechtheit aus. Nach längerem Belichten ändert sich die Nuance Ton-in-Ton.

Die gefärbten Papiere, die eine gute Bleichbarkeit aufweisen, sind naßecht, nicht nur gegen Wasser, sondern ebenfalls gegen Milch, Seifenwasser, Natriumchloridlösungen, Fruchtsäfte oder gesüßte Mineralwasser und wegen ihrer guten Alkoholechtheit auch gegen alkoholische Getränke beständig.

Mit den neuen Farbstoffen kann man auch Polyacrylnitriltextilien oder durch anionische Gruppen modifizierte Polyamid- oder Polyestertextilien färben, foulardieren oder bedrucken.

Die vorliegende Erfindung betrifft weiterhin neue Sulfonamide der Formel IIIa 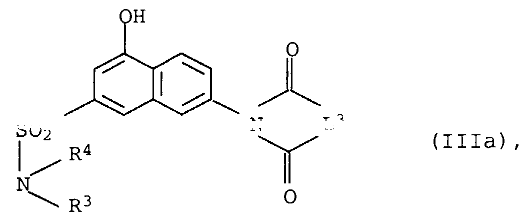 in der
L³ C₂-C₃-Alkylen, Vinylen oder 1,2-Phenylen und
- R³ und R⁴: gleich oder verschieden sind und unabhängig voneinander jeweils C₁-C₁₃-Alkyl, das gegebenenfalls durch 1 bis 4 Sauerstoffatome in Etherfunktion oder 1 bis 4 Iminogruppen, die durch C₁-C₄-Alkyl oder ω-Hydroxy-C₁-C₈-alkyl, das gegebenenfalls durch 1 bis 3 Sauerstoffatome in Etherfunktion unterbrochen ist, substituiert sein können, unterbrochen ist und durch Hydroxy, Amino, einen 5- oder 6-gliedrigen gesättigten oder aromatischen heterocyclischen Rest, der ein Stickstoffatom und gegebenenfalls ein weiteres Heteroatom, ausgewählt aus der Gruppe, bestehend aus Stickstoff, Sauerstoff und Schwefel, aufweist, oder gegebenenfalls substituiertes C₅-C₇-Cycloalkyl substituiert sein kann, gegebenenfalls substituiertes Phenyl, gegebenenfalls substituiertes C₅-C₇-Cycloalkyl oder gegebenenfalls durch Methyl substituiertes Piperidinyl oder R³ auch Wasserstoff bedeuten,
mit der Maßgabe, daß mindestens eine quaternierbare Aminogruppe im Molekül vorhanden ist.

Bevorzugt sind solche Sulfonamide der Formel IIIa, in der R³ Wasserstoff und R⁴ C₁-C₁₃-Alkyl, das mindestens ein quaternierbares Stickstoffatom aufweist, bedeuten.

Die folgenden Beispiele sollen die Erfindung näher erläutern. Die dort angegebenen Prozente sind Gewichtsprozente.

### Beispiel 1

30 g 1,2-Diaminopropan wurden bei 20°C mit 8,5 g (0,025 mol) N-Acetyl-I-Säurechlorid versetzt und 60 h bei Raumtemperatur gerührt. Das resultierende Öl wurde mit 200 ml Wasser verdünnt, mit Salzsäure aut einen pH-Wert von 8 gebracnt und bei einem Wert von pH 7,5 bis 8 mit (0,025 mol) diazotiertem p-Aminoacetanilid versetzt. Der pH-Wert wurde durch Zugabe von wäßriger Natriumcarbonatlösung konstant gehalten.
Die Lösung des Diazoniumsalzes wurde wie folgt hergestellt: 3,75 g (0,025 mol) p-Aminoacetanilid wurden bei 0 bis 3°C über 30 min mit 50 ml Wasser und 12,5 ml 30 gew.-%iger Salzsäure gerührt und anschließend mit 65 g Eis versetzt. Die Diazotierung erfolgte durch Zutropfen von 7,5 ml 23 gew.-%iger wäßriger Natriumnitritlösung. Das Reaktionsgemisch wurde weitere 2 h bei 0 bis 3°C gerührt und dann für die Kupplungsreaktion verwendet.

Nach Absaugen und Trocknen resultierten 9,5 g (76 %) Farbstoff der Formel 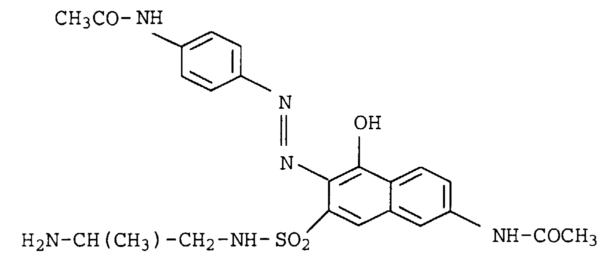 λmax: 502 nm (in 85 gew.%iger Essigsäure)

### Beispiel 2

3,08 g (0,025 mol) o-Anisidin wurden in 50 ml Wasser und 7 ml 38 gew.%iger Salzsäure gelöst, bei 0°C mit 17,3 ml 10 gew.%iger wäßriger Natriumnitritlösung versetzt, weitere 20 min bei 0°C gerührt und nach Zugabe von Aktivkohle klärfiltriert. Die resultierende Lösung des Diazoniumsalzes wurde, wie in Beispiel 1 beschrieben, mit einer äquimolaren Menge Sulfonamid umgesetzt.

Der Farbstoff der Formel 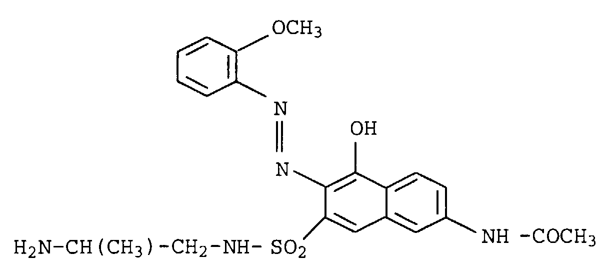 konnte in 62%iger Ausbeute (14,4 g) isoliert werden.
λmax: 501 nm (in 85 gew.%iger Essigsäure)

### Beispiel 3

In 30 g N,N-Dimethylethylendiamin wurden bei 20°C 8,5 g (0,025 mol) N-Acetyl-I-säurechlorid eingetragen und 60 h bei Raumtemperatur gerührt. Das resultierende Öl wurde mit 200 ml Wasser verdünnt, mit Salzsäure auf einen pH-Wert von 8 gebracht und bei einem pH-Wert von 7,5 bis 8 mit (0,025 mol) diazotiertem p-Aminoazobenzol gekuppelt. Der pH-Wert wurde durch Zugabe von wäßriger Natriumcarbonatlösung konstant gehalten.
Herstellung des Diazoniumsalzes des p-Aminoazobenzols: 4,93 g (0,025 mol) p-Aminoazobenzol wurden in 30 ml Wasser und 10 ml 30 gew.-%iger Salzsäure gelöst, 15 min bei Raumtemperatur gerührt, mit 150 g Eis/Wasser versetzt und bei 15°C auf einmal mit 7,5 ml 23 gew.-%iger wäßriger Natriumnitritlösung versetzt, weitere 2 h bei 15°C gerührt und anschließend klärfiltriert.

Die resultierende Lösung des Diazoniumsalzes wurde wie in Beipsiel 1 beschrieben mit einer äquimolaren Menge Sulfonamid umgesetzt.

Nach Absaugen und Trocknen resultierten 19,4 g (70 %) Farbstoff der Formel 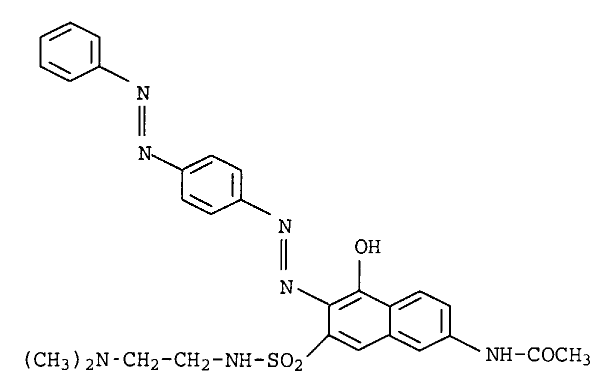 λmax: 506 nm (in 85 gew.%iger Essigsäure)

In analoger Weise werden die in der folgenden Tabelle aufgeführten Farbstoffe der Formel 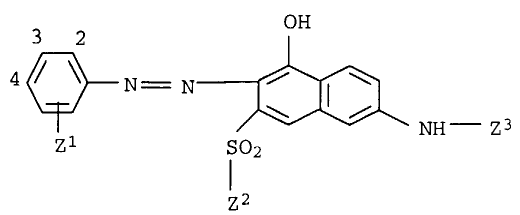 erhalten.

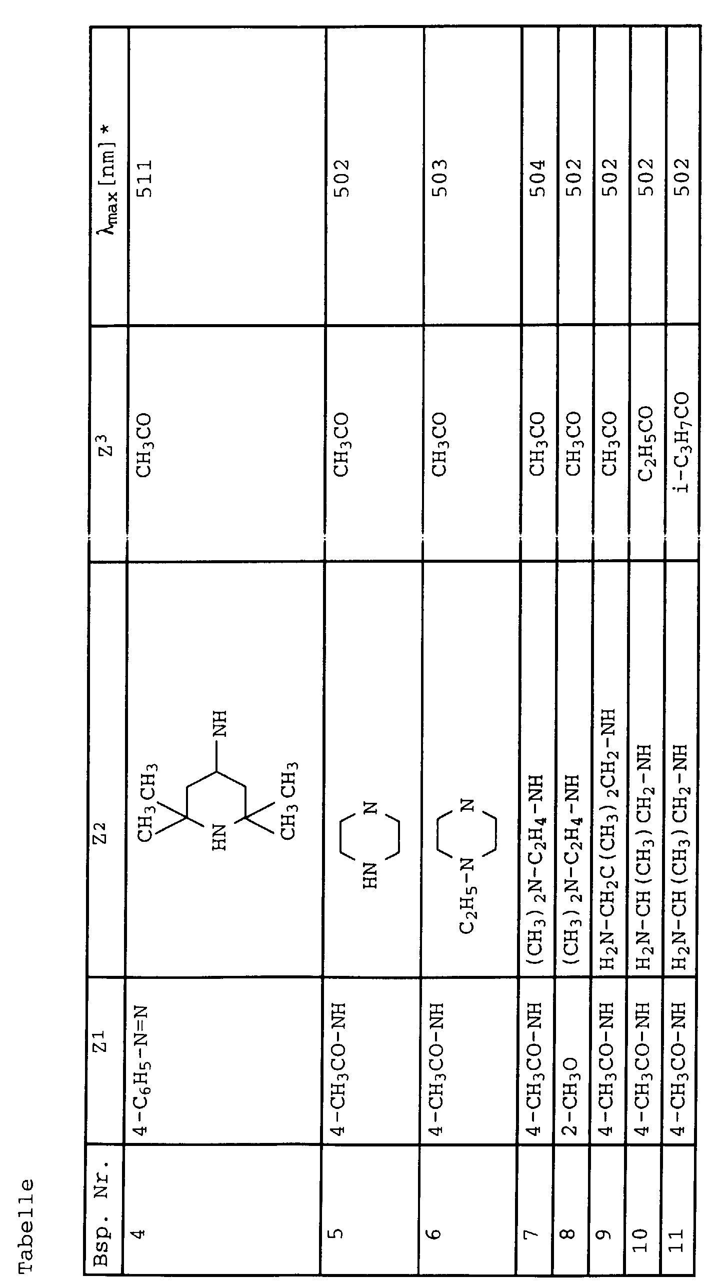

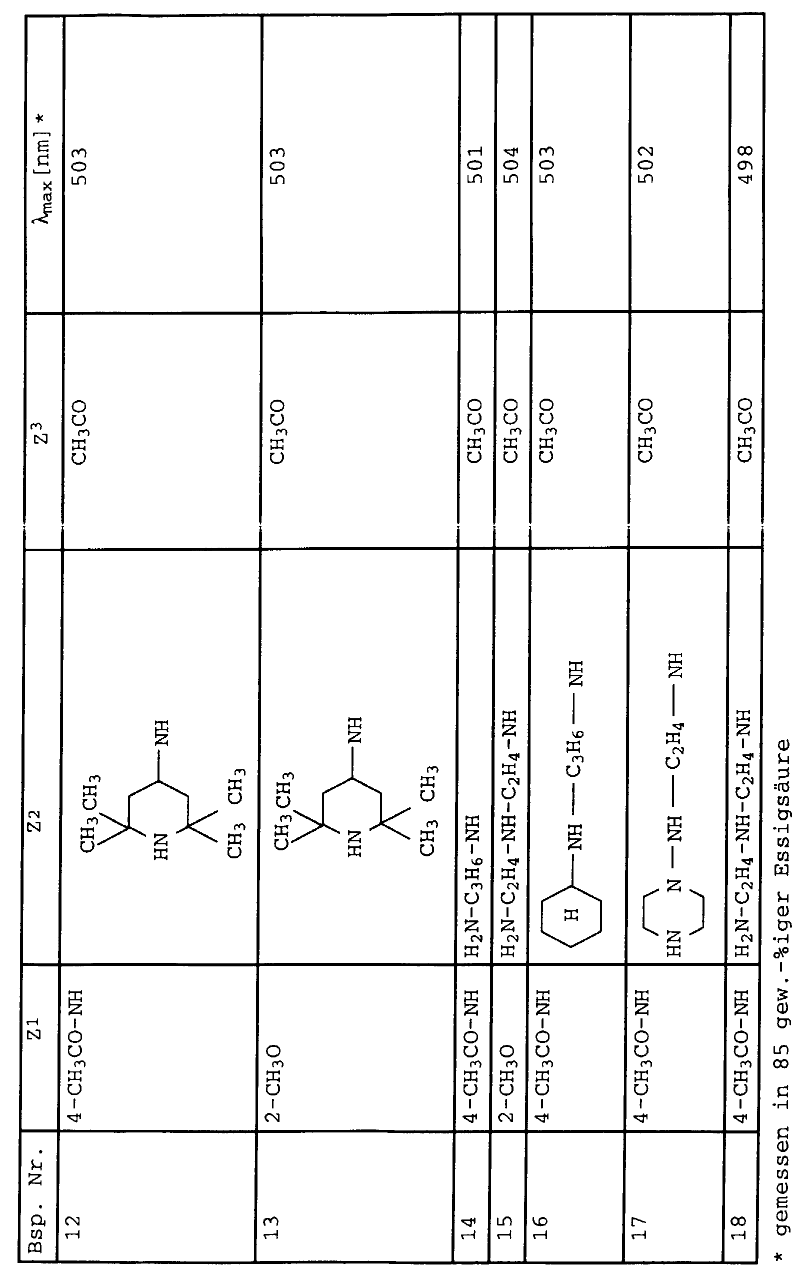

### Beispiel 19

8,2 g (0,05 mol) N-Acetyl-N-methyl-p-phenylendiamin wurden in einer Mischung aus 100 g Eis, 125 ml 30 gew.-%iger Salzsäure und 12,5 ml Wasser gelöst. Nach der Zugabe von 12,5 g Eis wurde bei 0 bis 5°C mit 15 ml 23 gew.-%iger wäßriger Natriumnitritlösung versetzt, eine weitere Stunde bei 0 bis 5°C gerührt und anschließend klärfiltriert.

Die resultierende Lösung des Diazoniumsalzes wurde, wie in Beispiel 1 beschrieben, mit einer äquimolaren Menge Sulfonamid umgesetzt.

Nach Absaugen und Trocknen resultierten 17 g (66 %) Farbstoff der Formel 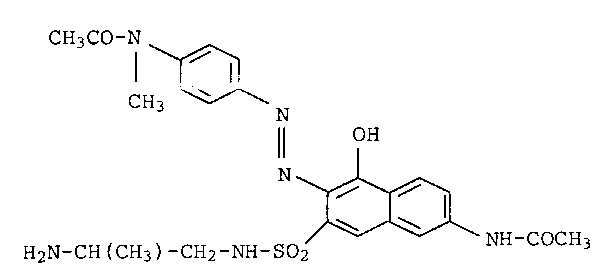 λmax: 488 nm (in 85 gew.%iger Essigsäure)

### Beispiel 20

382 g (1,6 mol) I-Säure wurden in 1000 g Wasser und 1000 g Aceton gelöst und mit 90 ml 50 gew.-%iger Natronlauge auf einen pH-Wert von 7 gestellt. Innerhalb von 3h wurden bei 0 bis 5°C 339 g (3 mol) Chloracetylchlorid zugetropft, wobei der pH-Wert mittels 20 gew.-%iger wäßriger Sodalösung auf 6,1 gehalten wurde. Anschließend wurde Aceton und Wasser unter vermindertem Druck bis auf ein Rückstandsvolumen von 1000 ml abgezogen. Nach Zugabe von 500 ml Wasser wurde mit konz. Salzsäure ein pH-Wert von 2,7 eingestellt und die ausgefallene Chloracetyl-I-Säure isoliert.

66,8 g (0,2 mol) der nach der obigen Vorschrift erhaltenen Chloracetyl-I-Säure wurden bei 25 bis 30°C in 104 ml Phosphoroxidtrichlorid und 40 ml N,N-Dimethylacetamid eingetragen und weitere 18h bei dieser Temperatur belassen.

Nach Fällen in Eiswasser wurde das Sulfonsäurechlorid isoliert, in Essigester aufgenommen über Natriumsulfat getrocknet und zur Trockene einrotiert.

13,4 g (0,04 mol) Chloracetyl-I-Säurechlorid wurden bei 25 bis 30°C in 50 g Ethanolamin eingetragen, 18 h bei Raumtempertur gerührt, auf 250 g Eis gegeben und mit 70 g 30 gew.-%iger Salzsäure auf einen pH-Wert von 7,5 gestellt. Die Lösung wurde, wie in Beispiel 1 beschrieben, mit 0,04 mol diazotiertem p-Aminoacetanilid gekuppelt.
Ausbeute: 18,2 g (82 %) Farbstoff der Formel 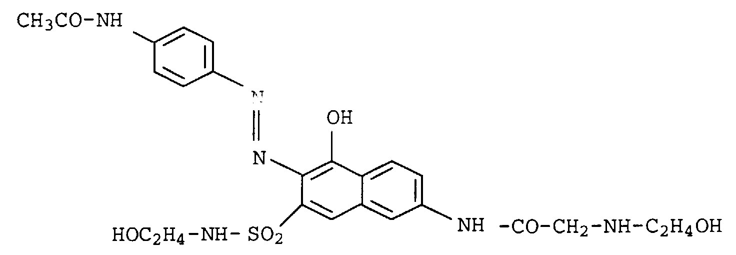 λmax: 488 nm (in 85 gew.%iger Essigsäure)

### Beispiel 21

16,7 g (0,05 mol) Chloracetyl-I-Säurechlorid wurden bei 25 bis 30°C in 60 g 1,2-Diaminopropan eingetragen, 18 h bei Raumtemperatur gerührt, auf 250 g Eis gegeben und mit 70 g 30 gew.-%iger Salzsäure auf einen pH-Wert von 7,5 gestellt. Die Lösung wurde, wie in Beispiel 1 beschrieben, mit 0,05 mol diazotiertem p-Aminoacetanilid gekuppelt.
Ausbeute: 17 g (54 %) Farbstoff der Formel 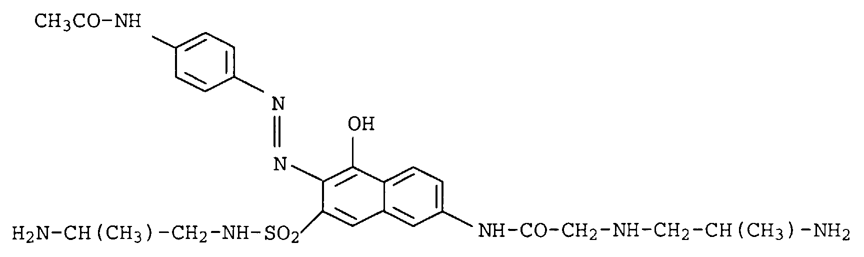 λmax: 499 nm (in 85 gew.%iger Essigsäure)

### Beispiel 22

20 g (80,2 mol) Bernsteinsäureanhydrid wurden in 50 g N,N-Dimethylacetamid gelöst und bei 50°C mit 23,8 g (0,1 mol) I-Säure versetzt. Nach weiteren 1,5 h bei 70°C wurde auf 30°C abgekühlt, mit 76,7 g (0,5 mol) Phosphoroxidtrichlorid versetzt und 18 h bei Raumtemperatur gerührt. Die Isolierung des Sulfonsäurechlorids erfolgte durch Hydrolyse in Eiswasser, Absaugen des Feststoffes und anschließendes Aufnehmen in Essigester. Nach Trocknen über Natriumsulfat und Entfernen des Lösungsmittels unter vermindertem Druck resultierten 31,5 g (93 %) Sulfonsäurechlorid.

Das Sulfonsäurechlorid wurde, wie in Beispiel 1 beschrieben, zunächst in das Amid und dann in den Farbstoff übergeführt.

Durch Aussalzen konnten 40,3 g (99 %) Farbstoff der Formel 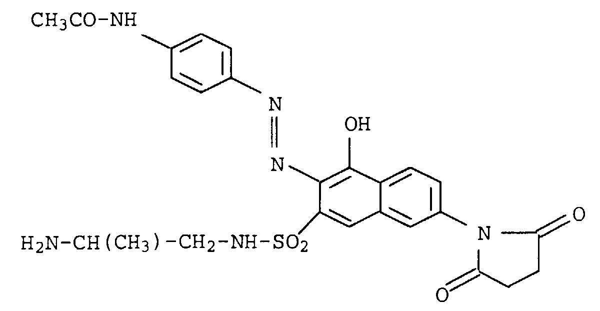 isoliert werden.
λmax: 502 nm (in 85 gew.%iger Essigsäure).

## Patentansprüche

1. Farbstoffe der Formel I 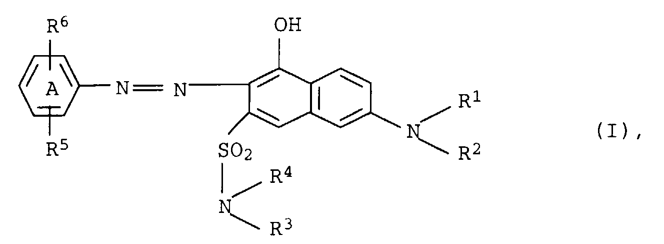 in der
der Ring A benzoanelliert sein kann,
R¹ C₁-C₁₃-Alkyl, Benzyl, 2-Chlorethyl, gegebenenfalls substituiertes Phenyl, C₁-C₈-alkonoyl, gegebenenfalls substituiertes Benzoyl oder einen Rest der Formel 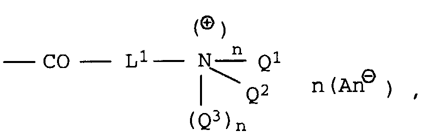 worin L¹ für C₁-C₃-Alkylen,
n für 0 oder 1,
Q¹, Q², Q³ gleich oder verschieden sind und unabhängig voneinander jeweils für Wasserstoff oder gegebenenfalls durch Hydroxy substituiertes C₁-C₈-Alkyl, das durch 1 bis 3 Sauerstoffatome in Etherfunktion unterbrochen sein kann, oder Q¹ und Q² zusammen mit dem sie verbindenden Stickstoffatom für einen 5- oder 6-gliedrigen gesättigten heterocyclischen Rest, der gegebenenfalls Stickstoff oder Sauerstoff als weiteres Heteroatom aufweist, und
An^{⊖} für das Äquivalent eines Anions stehen,
R² Wasserstoff oder C₁-C₄-Alkyl oder R¹ und R² zusammen einen Rest der Formel
CO-L² oder CO-L³-CO, worin
L² für C₃-C₄-Alkylen und
L³ für C₂-C₃-Alkylen, Vinylen oder 1,2-Phenylen stehen,
R³ und R⁴ gleich oder verschieden sind uns unabhängig voneinander jeweils C₁-C₁₃-Alkyl, das gegebenenfalls durch 1 bis 4 Sauerstoffatome in Etherfunktion oder 1 bis 4 Iminogruppen, die durch C₁-C₄-Alkyl oder ω-Hydroxy-C₁-C₈-alkyl, das gegebenenfalls durch 1 bis 3 Sauerstoffatome in Etherfunktion unterbrochen ist, substituiert sein können, unterbrochen ist und durch Hydroxy,Amino, einen 5- oder 6-gliedrigen gesättigten oder aromatischen heterocyclischen Rest, der ein Stickstoffatom und gegebenenfalls ein weiteres Heteroatom, ausgewählt aus der Gruppe, bestehend aus Stickstoff, Sauerstoff und Schwefel, aufweist, oder gegebenenfalls substituiertes C₅-C₇-Cycloalkyl substituiert sein kann, gegebenenfalls substituiertes Phenyl, gegebenenfalls substituiertes C₅-C₇-Cycloalkyl oder gegebenenfalls durch Methyl substituiertes Piperidinyl oder R³ auch Wasserstoff,
R⁵ Wasserstoff, C₁-C₄-Alkyl, C₁-C₄-Alkoxy, C₂-C₅-Alkanoylamino, Halogen, Nitro, Cyano, gegebenenfalls durch C₁-C₄-Alkyl substituiertes Phenylazo oder einen Rest der Formel 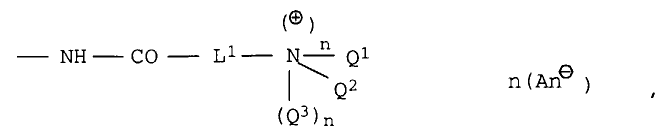 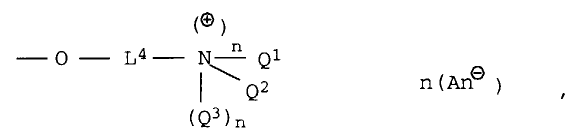 oder 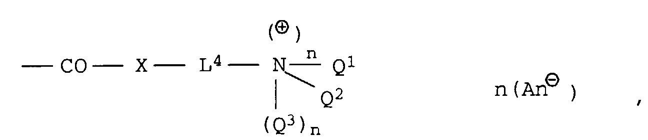 worin X für Sauerstoff oder Imino und L⁴ für C₂-C₄-Alkylen stehen und Q¹, Q², Q³, n und An^{⊖} jeweils die obengenannte Bedeutung besitzen, und
R⁶ Wasserstoff, C₁-C₄-Alkyl, C₁-C₄-Alkoxy, Halogen, Nitro oder Cyano bedeuten,
mit der Maßgabe, daß mindestens eine quaternierbare Aminogruppe oder eine Ammoniumgruppe im Molekül vorhanden ist.

2. Farbstoffe nach Anspruch 1, dadurch gekennzeichnet, daß R¹ C₂-C₄-Alkanoyl, Benzoyl oder einen Rest der Formel 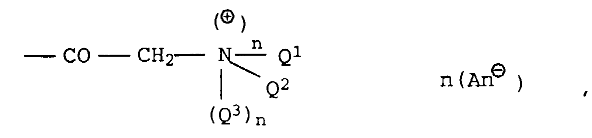 worin Q¹, Q², Q³, An^{⊖} und n jeweils die in Anspruch 1 genannte Bedeutung besitzen, oder R¹ und R² zusammen einen Rest der Formel CO-L³-CO, worin L³ für Ethylen, Vinylen oder 1,2-Phenylen steht, und
R² Wasserstoff oder C₁-C₄-Alkyl bedeuten.

3. Farbstoffe nach Anspruch 1, dadurch gekennzeichnet, daß
R³ Wasserstoff und
R⁴ C₁-C₁₃-Alkyl bedeuten, das durch 1 oder 2 Sauerstoffatome in Etherfunktion, Imino- oder C₁-C₄-Al-kyliminogruppen unterbrochen ist und/oder durch Amino oder einen 5- oder 6-gliedrigen gesättigten oder aromatischen heterocyclischen Rest, der ein oder zwei Stickstoffatome aufweist, substituiert ist.

4. Farbstoffe nach Anspruch 1, dadurch gekennzeichnet, daß
R⁵ C₁-C₄-Alkyl, C₁-C₄-Alkoxy, C₂-C₄-Alkanoylamino, oder Phenylazo und
R⁶ Wasserstoff bedeuten.

5. Verwendung der Farbstoffe gemäß Anspruch 1 zum Färben oder Bedrucken von polymerem Material.

6. Sulfonamide der Formel IIIa 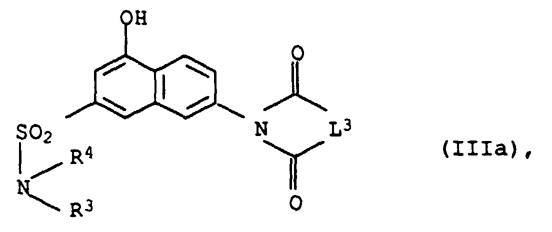 in der
L³ C₂-C₃-Alkylen, Vinylen oder 1,2-Phenylen und
R³ und R⁴ gleich oder verschieden sind und unabhängig voneinander jeweils C₁-C₁₃-Alkyl, das gegebenenfalls durch 1 bis 4 Sauerstoffatome in Etherfunktion oder 1 bis 4 Iminogruppen, die durch C₁-C₄-Alkyl oder ω-Hydroxy-C₁-C₈-alkyl, das gegebenenfalls durch 1 bis 3 Sauerstoffatome in Etherfunktion unterbrochen ist, substituiert sein können, unterbrochen ist und durch Hydroxy,Amino, einen 5- oder 6-gliedrigen gesättigten oder aromatischen heterocyclischen Rest, der ein Stickstoffatom und gegebenenfalls ein weiteres Heteroatom, ausgewählt aus der Gruppe, bestehend aus Stickstoff, Sauerstoff und Schwefel, aufweist, oder gegebenenfalls substituiertes C5-C7-Cycloalkyl substituiert sein kann, gegebenenfalls substituiertes Phenyl, gegebenenfalls substituiertes C5-C7-Cycloalkyl oder gegebenenfalls durch Methyl substituiertes Piperidinyl oder R3 auch Wasserstoff bedeuten,
mit der Maßgabe, daß mindestens eine quaternierbare Aminogruppe im Molekül vorhanden ist.

## Claims

1. Dyes of the formula I 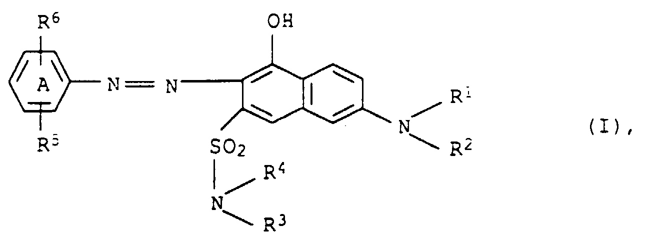 where
the ring A may be benzofused,
R¹ is C₁-C₁₃-alkyl, benzyl, 2-chloroethyl, substituted or unsubstituted phenyl, C₁-C₈-alkanoyl, substituted or unsubstituted benzoyl or a radical of the formula 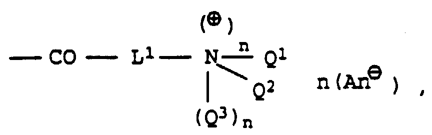 where L¹ is C₁-C₃-alkylene,
n is 0 or 1,
Q¹, Q² and Q³ are identical or different and each is independently of the others hydrogen or unsubstituted or hydroxyl-substituted C₁-C₈-alkyl, which may be interrupted by from 1 to 3 oxygen atoms in ether function, or Q¹ and Q², together with the nitrogen atom joining them together, are a 5- or 6-membered saturated heterocyclic radical which may contain nitrogen or oxygen as further hetero atom, and
An^{⊖} is the equivalent of an anion,
R² is hydrogen or C₁-C₄-alkyl or R¹ and R² together are a radical of the formula
CO-L² or CO-L³-CO, where
L² is C₃-C₄-alkylene and
L³ is C₂-C₃-alkylene, vinylene or 1,2-phenylene,
R³ and R⁴ are identical or different and each is independently of the other C₁-C₁₃-alkyl, which may be interrupted by from 1 to 4 oxygen atoms in ether function or by from 1 to 4 imino groups which may be substituted by C₁-C₄-alkyl or ω-hydroxy-C₁-C₈-alkyl, which may be interrupted by from 1 to 3 oxygen atoms in ether function, and may be substituted by hydroxyl, by amino, by a 5- or 6-membered saturated or aromatic heterocyclic radical which contains a nitrogen atom and may contain a further hetero atom selected from the group consisting of nitrogen, oxygen and sulfur, or by substituted or unsubstituted C₅-C₇-cycloalkyl, substituted or unsubstituted phenyl, substituted or unsubstituted C₅-C₇-cycloalkyl or unsubstituted or methyl-substituted piperidinyl, and R³ may also be hydrogen,
R⁵ is hydrogen, C₁-C₄-alkyl, C₁-C₄-alkoxy, C₂-C₅-alkanoylamino, halogen, nitro, cyano, unsubstituted or C₁-C₄,-alkyl-substituted phenylazo or a radical of the formula 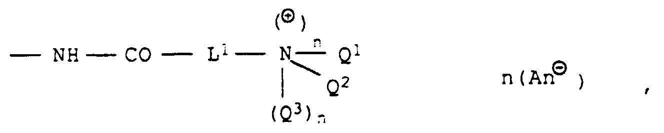 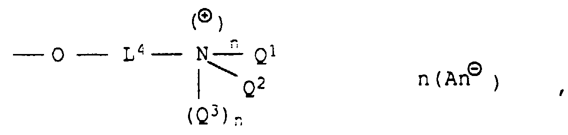 or 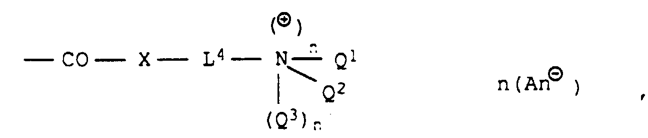 where X is oxygen or imino, and L⁴ is C₂-C₄-alkylene, and Q¹, Q², Q³, n and An^{⊖} are each as defined above, and
R⁶ is hydrogen, C₁-C₄-alkyl, C₁-C₄-alkoxy, halogen, nitro or cyano,
with the proviso that at least one quaternizable amino group or an ammonium group be present in the molecule.

2. Dyes as claimed in claim 1, wherein
R¹ is C₂-C₄-alkanoyl, benzoyl or a radical of the formula 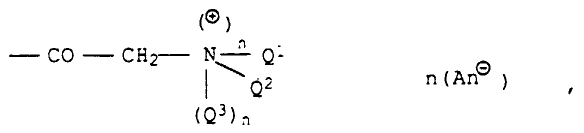 where Q¹, Q², Q³, An^{⊖} and n are each as defined in claim 1, or R¹ and R² are together a radical of the formula CO-L³-CO, where L³ is ethylene, vinylene or 1,2-phenylene, and
R² is hydrogen or C₁-C₄-alkyl.

3. Dyes as claimed in claim 1, wherein
R³ is hydrogen and
R⁴ is C₁-C₁₃-alkyl which is interrupted by 1 or 2 oxygen atoms in ether function, imino or C₁-C₄-alkylimino groups and/or substituted by amino or by a 5- or 6-membered saturated or aromatic heterocyclic radical which contains one or two nitrogen atoms.

4. Dyes as claimed in claim 1, wherein
R⁵ is C₁-C₄-alkyl, C₁-C₄-alkoxy, C₂-C₄-alkanoykamino or phenylazo and
R⁶ is hydrogen.

5. The use of the dyes of claim 1 for dyeing or printing polymeric material.

6. Sulfonamides of the formula IIIa 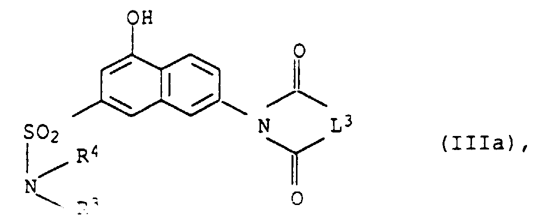 where
L³ is C₂-C₃-alkylene, vinylene or 1,2-phenylene and
R³ and R⁴ are identical or different and each is independently of the other C₁-C₁₃-alkyl, which may be interrupted by from 1 to 4 oxygen atoms in ether function or by from 1 to 4 imino groups which may be substituted by C₁-C₄-alkyl or ω-hydroxy-C₁-C₈-alkyl, which may be interrupted by from 1 to 3 oxygen atoms in ether function, and may be substituted by hydroxyl, by amino, by a 5- or 6-membered saturated or aromatic heterocyclic radical which contains a nitrogen atom and may contain a further hetero atom selected from the group consisting of nitrogen, oxygen and sulfur, or by substituted or unsubstituted C₅-C₇-cycloalkyl, substituted or unsubstituted phenyl, substituted or unsubstituted C₅-C₇-cycloalkyl or unsubstituted or methyl-substituted piperidinyl, and R³ may also be hydrogen,
with the proviso that at least one quaternizable amino group be present in the molecule.

## Revendications

1. Colorants de formule I 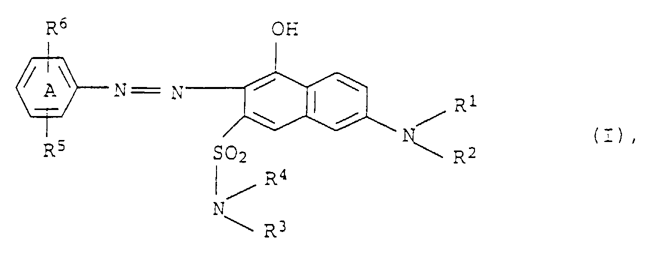 dans laquelle
le noyau A peut être benzo-condensé,
R¹ est un groupe alkyle en C₁-C₁₃, benzyle, 2-chloroéthyle, phényle éventuellement substitué, alcanoyle en C₁-C₈, benzoyle éventuellement substitué, ou encore un radical de formule 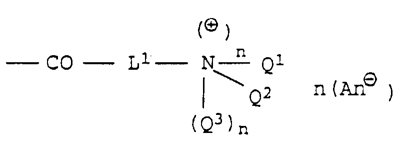 dans laquelle L¹ est un groupe alkylène en C₁-C₃,
n vaut 0 ou 1,
Q¹, Q² et Q³ sont identiques ou différents et représentent chacun indépendamment des autres un atome d'hydrogène ou un groupe alkyle en C₁-C₈ éventuellement hydroxylé, qui peut être interrompu par 1 à 3 atomes d'oxygène en fonction éther, ou encore Q¹ et Q², avec l'atome d'azote qui les relie, représentent un radical hétérocyclique saturé ayant 5 ou 6 chaînons, qui comporte éventuellement un atome d'azote ou d'oxygène en tant qu'hétéroatome supplémentaire, et
An⁻ est l'équivalent d'un anion,
R² est un hydrogène ou un groupe alkyle en C₁-C₄, ou bien R¹ et R² forment ensemble un groupe de formule CO-L² ou CO-L³-CO, où L² est un groupe alkylène en C₃-C₄, et L³ est un groupe alkylène en C₂-C₃, vinylène ou 1,2-phénylène,
R³ et R⁴ sont identiques ou différents et représentent chacun indépendamment de l'autre un groupe alkyle en C₁-C₁₃, qui est éventuellement interrompu par 1 à 4 atomes d'oxygène en fonction éther ou par 1 à 4 groupes imino, qui peuvent être substitués par des substituants alkyle en C₁-C₄ ou ω-hydroxy-(alkyle en C₁-C₈), qui sont éventuellement interrompus par 1 à 3 atomes d'oxygène en fonction éther, et qui peut être substitué par des substituants hydroxy, amino, par un radical hétérocyclique saturé ou aromatique ayant 5 ou 6 chaînons, qui comportent un atome d'azote et éventuellement un autre hétéroatome, choisi dans l'ensemble constitué de l'azote, de l'oxygène et du soufre, ou encore car un groupe cycloalkyle en C₅-C₇ éventuellement substitué ; phényle éventuellement substitué, cycloalkyle en C₅-C₇ éventuellement substitué, ou pipéridinyle éventuellement substitué par des groupes méthyle, ou encore R³ peut aussi être un hydrogène,
R⁵ est un hydrogène ou un groupe alkyle en C₁-C₄, alcoxy en C₁-C₄, alcanoylamino en C₂-C₅, halogéno, nitro, cyano, phénylazo éventuellement substitué par des groupes alkyle en C₁-C₄, ou encore un radical de formule 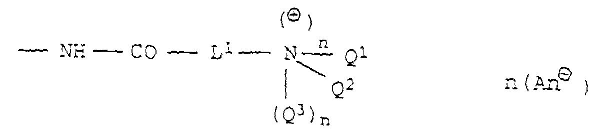 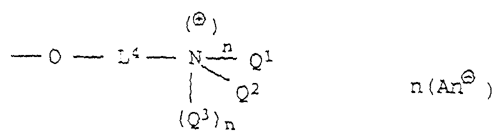 ou 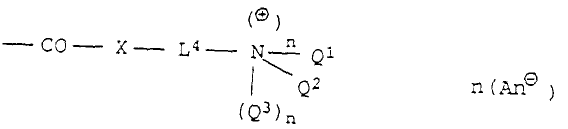 où X est un oxygène ou le groupe imino, et L⁴ est un groupe alkylène en C₂-C₄, et Q¹, Q², Q³, n et An⁻ ont chacun les significations données ci-dessus, et
R⁶ est un hydrogène ou un groupe alkyle en C₁-C₄, alcoxy en C₁-C₄, halogéno, nitro ou cyano,
du moment qu'au moins un groupe amino quaternisable ou un groupe ammonium est présent dans la molécule.

2. Colorants selon la revendication 1, caractérisés en ce que R¹ est un groupe alcanoyle en C₂-C₄, benzoyle, ou un radical de formule 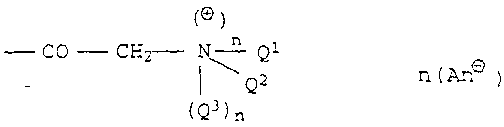 dans laquelle Q¹, Q², Q³, An⁻ et n ont chacun les significations données dans la revendication 1, ou bien R¹ et R² forment ensemble un radical de formule CO-L³-CO, dans laquelle L³ est le groupe éthylène, vinylène ou 1,2-phénylène, et
R² est un hydrogène ou un groupe alkyle en C₁-C₄.

3. Colorants selon la revendication 1, caractérisés en ce que
R³ est un hydrogène, et
R⁴ est un groupe alkyle en C₁-C₁₃, qui est interrompu par 1 ou 2 atomes d'oxygène en fonction éther, imino ou alkylimino en C₁-C₄, et/ou est substitué par des substituants amino ou un radical hétérocyclique saturé ou aromatique ayant 5 ou 6 chaînons, qui présentent 1 ou 2 atomes d'azote.
4. Colorants selon la revendication 1, caractérisés en ce que
R⁵ est un groupe alkyle en C₁-C₄, alcoxy en C₁-C₄, alcanoylamino en C₂-C₄ ou phénylazo, et
R⁶ est un hydrogène.
5. Utilisation des colorants selon la revendication 1, pour teindre ou imprimer un matériau polymère.
6. Sulfonamides de formule IIIa 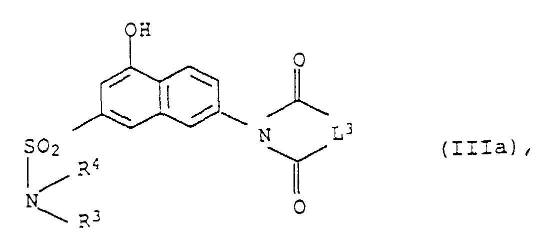 dans laquelle
L³ est un groupe alkylène en C₂-C₃, vinylène ou 1,2phénylène, et
R³ et R⁴ sont identiques ou différents et représentent chacun indépendamment de l'autre un groupe alkyle en C₁-C₁₃, qui est éventuellement interrompu par 1 à 4 atomes d'oxygène en fonction éther ou par 1 à 4 groupes imino, qui peuvent être substitués par des substituants alkyle en C₁-C₄ ou ω-hydroxy-(alkyle en C₁-C₈), qui sont éventuellement interrompus par 1 à 3 atomes d'oxygène en fonction éther, et qui peut être substitué par des substituants hydroxy, amino, par un radical hétérocyclique saturé ou aromatique ayant 5 ou 6 chaînons, qui comportent un atome d'azote et éventuellement un autre hétéroatome, choisi dans l'ensemble constitué de l'azote, de l'oxygène et du soufre, ou encore par un groupe cycloalkyle en C₅-C₇ éventuellement substitué ; phényle éventuellement substitué, cycloalkyle en C₅-C₇ éventuellement substitué, ou pipéridinyle éventuellement substitué par des groupes méthyle, ou encore R³ peut aussi être un hydrogène,
du moment qu'au moins un groupe amino quaternisable est présent dans la molécule.
